# EUROPEAN PATENT SPECIFICATION

(11) **EP 4 209 558 B1**
(45) Date of publication and mention of the grant of the patent: **09.12.2026**
(21) Application number: 21864187.6
(22) Date of filing: 24.08.2021
(51) Int. Cl.: C09D 11/328, C09D 11/38, A61K 9/44, A61K 47/12, A61K 47/14, A61K 47/22

(54) **EDIBLE INK-JET INK AND TABLET**
ESSBARE TINTENSTRAHLTINTE UND TABLETTE
ENCRE JET D'ENCRE COMESTIBLE ET COMPRIMÉ

(30) Priority: 04.09.2020 JP 2020149381
(43) Date of publication of application: 12.07.2023
(73) Proprietor: TOPPAN INC., Tokyo 110-0016 (JP)
(72) Inventor: KOSEKI, Seiya, Tokyo 110-0016 (JP); ISHIKAWA, Hideki, Tokyo 110-0016 (JP); HOSHINO, Yuichi, Tokyo 110-0016 (JP); SAITO, Masatoshi, Tokyo 110-0016 (JP)
(74) Representative: TBK
(86) International application number: PCT/JP2021/030985
(87) International publication number: WO 2022/050129

(56) References cited:
- WO-A1-2018/061848
- JP-A- 2015 091 647
- JP-A- 2015 091 647
- JP-A- 2019 123 840
- JP-A- 2019 127 589
- JP-A- S57 179 263
- US-A1- 2009 182 052

## Description

### [Technical Field]

The present invention relates to edible inkjet inks and tablets.

### [Background Art]

Inkjet printing inks (which may also be simply referred to as IJ ink(s) hereinafter) include those inks which are edible with use of food dyes, such as tar pigments, as colorants (which may also be simply referred to as edible IJ ink(s) hereinafter).

Edible IJ inks based on the conventional art may cause discoloration, i.e., change of color (hue) of colorants in printed characters, images, and the like (which are collectively referred to as printed image(s) hereinafter) due to deterioration of the colorants, or may cause fading due to desaturation or the like of the colorants.

For example, when printing images on printing targets such as tablets, chromogenic dyes have been used as colorants of edible IJ inks (dye inks) for the purpose of improving visibility. However, since dyes are deteriorated by being decomposed by the action of light (photolyzed), discoloration or fading may occur in the printed images due to light.

To suppress discoloration or fading of such printed images in recent years, technologies are being developed, and various methods to suppress discoloration or fading have been proposed (e.g., see PTL 1). Additionally, JP 2019123840 might be cited as prior art. However, methods based on the conventional art have been insufficient for suppressing photodiscoloration or photofading of printed images, and thus lightfastness of the edible IJ inks could not be improved.

### [Citation List]

### [Patent Literature]

PTL 1: JP 6389506 B

### [Summary of the Invention]

### [Technical Problem]

The present invention has been made in light of the circumstances described above and aims to provide an edible IJ ink which can sufficiently suppress photodiscoloration or photofading of printed images and improve lightfastness, and to provide a tablet including a printed part that is printed using the edible IJ ink.

### [Solution to Problem]

In order to accomplish the above aim, an edible IJ ink according to an aspect of the present invention contains Red No. 3, and hydroxy acid salt having two or more carboxyl groups.

Furthermore, in order to accomplish the above aim, a tablet according to an aspect of the present invention includes a printed part that is printed using the edible IJ ink.

### [Advantageous Effects of the Invention]

According to an aspect of the present invention, photodiscoloration or photofading of printed images can be sufficiently suppressed, and lightfastness of the edible IJ ink can be improved.

### [Brief Description of the Drawings]

Fig. 1 is a schematic cross-sectional view illustrating an example of a tablet (uncoated tablet) according to an embodiment of the present invention.
Fig. 2 is a schematic cross-sectional view illustrating an example of a tablet (film-coated tablet) according to an embodiment of the present invention.
Fig. 3 is a diagram illustrating an example of a printed image for a tablet (uncoated tablet) according to an embodiment of the present invention.
Fig. 4 is a diagram illustrating an example of a printed image for a tablet (film-coated tablet) according to an embodiment of the present invention.

### [Description of the Embodiments]

An IJ ink according to an embodiment of the present invention relates to edible IJ inks which can sufficiently suppress photodiscoloration or photofading and improve lightfastness of printed images provided on, for example, the surfaces of pharmaceutical tablets and the like using inkjet ink printing methods. The following description explains in detail the composition of an edible IJ ink according to an embodiment of the present invention and a tablet including a printed part that is printed using the edible IJ ink.

### [Composition of edible IJ ink]

The edible IJ ink of the present embodiment contains Food Red No. 3 (hereinafter simply referred to as Red No. 3) and hydroxy acid salt having two or more carboxyl groups Although the details will be described later, the hydroxy acid salt in the edible IJ ink of the present embodiment functions as a discoloration inhibitor that suppresses photodiscoloration or photofading of Red No. 3. With this composition, photodiscoloration or photofading of printed images can be sufficiently suppressed, and lightfastness of the edible IJ ink can be improved.

Advantageous effects achieved by the edible IJ ink of the present embodiment will be described.

Among various colorants used for edible IJ inks, tar pigments, for example, used as food dyes can cause discoloration or fading in the characters, images, and the like printed on the surfaces such as of solid formulations.

For example, of the tar pigments, Red No. 3 has caused photodiscoloration or photofading due to photolysis in the printed images, such as characters and images, printed on the surfaces of solid formulations, etc. Despite the development of technologies to suppress discoloration or fading of printed images, discoloration inhibitors (tea extract, vitamin C, etc.) of the conventional art have not been able to sufficiently suppress photolysis of Red No. 3.

In this regard, the edible IJ ink according to the present embodiment can suppress photolysis of Red No. 3 to sufficiently suppress photodiscoloration or photofading of printed images and improve lightfastness, by selecting types or physical properties of discoloration inhibitors. The present inventors have found that, by adding hydroxy acid salt having two or more carboxyl groups as a discoloration inhibitor to an ink containing a specific tar pigment, i.e., Red No. 3, as a colorant, a specific lightfastness-enhancing effect could be expressed and photolysis of Red No. could be suppressed. Thus, the present inventors have achieved sufficient suppression of photodiscoloration or photofading of printed images and improvement of lightfastness of an edible IJ ink by formulating the edible IJ ink to contain Red No. 3, and hydroxy acid salt having two or more carboxyl groups.

Hereinafter, components composing the edible IJ ink of the present embodiment will be described.

### (Colorant)

As described above, the edible IJ ink of the present embodiment contains Red No. 3 as a colorant. It should be noted that Red No. 3 (FDA Name: FD & C Red No. 3, Color Index Name: Acid Red 51, CAS Number: 16423-68-0 ) is a food dye which is specifically a food tar pigment also called erythrosine. In the edible IJ ink of the present embodiment, the blending ratio of Red No. 3, i.e., the content of Red No. 3, is preferred to be in the range of 2.5 mass% or more and 10 mass% or less with respect to the total mass of the ink. With this composition, undissolved residue of Red No. 3 decreases when producing the edible IJ ink, while good visibility can be imparted to printed images. Herein, for example, if undissolved residue cannot be visually recognized after the step of stirring a solvent and Red No. 3 (e.g., the step of adding Red No. 3 to a solvent at 25°C and stirring the mixture for 60 minutes) when producing the edible IJ ink, solubility of Red No. 3 in the solvent is assumed to be secured.

In contrast, if the total content of the Red No. 3 is less than 2.5 mass%, the overall printed color tends to be lighter and thus visibility of the printed image tends to be deteriorated. If the content exceeds 10 mass%, undissolved residue of Red No. 3 could be left in the edible IJ ink in the stirring step when producing the ink.

It should be noted that the edible IJ ink of the present embodiment may contain pigments (colorants) other than the specific tar pigment, i.e., Red No. 3. There is no particular limitation in the pigments other than Red No. 3, as long as they are edible. Pigments that can be added to the edible IJ ink of the present embodiment can be appropriately selected from, for example, known synthetic and natural food pigments. If the edible IJ ink contains colorants other than Red No. 3, the total content of the colorants may only need to be in the above range (2.5 mass% or more and 10 mass% or less). Thus, sufficient visibility can be imparted to printed images, and undissolved residue of the colorants can be prevented from remaining in the edible IJ ink.

The synthetic food pigments include, for example, tar pigments, natural pigment derivatives, natural synthetic pigments, and the like. Examples of the tar pigments include Food Red No. 2 (Amaranth, FDA Name: FD & C Red No. 2, Color Index Name: Acid Red 27, CAS Number: 915-67-3), Food Red No. 40 (Allura Red AC, FDA Name: FD & C Red No. 40, Color Index Name: Food Red No. 40, CAS Number: 25956-17-6), Food Red No. 102 (New Coccine, Color Index Name: Acid Red 18, CAS Number: 2611-82-7), Food Red No. 104 (Phloxine, FDA Name: FD & C Red No. 28, Color Index Name: Acid Red 92, CAS Number: 18472-87-2), Food Red No. 105 (Rose Bengal, Color Index Name: Acid Red 94, CAS Number: 632-69-9), Food Red No. 106 (Acid Red, Color Index Name: Acid Red 52, CAS Number: 3520-42-1) Food Yellow No. 4 (Tartrazine, FDA Name: FD & C Yellow No. 5, Color Index Name: Acid Yellow 23, CAS Number: 1934-21-0), Food Yellow No. 5 (Sunset Yellow FCF, FDA Name: FD & C Yellow No. 6, Color Index Name: Food Yellow 3, CAS Number: 2783-94-0), Food Blue No. 1 (Brilliant Blue FCF, FDA Name: FD & C Blue No. 1, Color Index Name: Food Blue 2, CAS Number: 3844-45-9), Food Blue No. 2 (Indigo Carmine, FDA Name: FD & C Blue No. 2, Color Index Name: Acid Blue 74, CAS Number: 860-22-0), Food Red No. 2 Aluminum Lake (FD & C Red No. 2 Aluminum Lake), Food Red No. 3 Aluminum Lake (FD & C Red No. 3 Aluminum Lake), Food Red No. 40 Aluminum Lake (FD & C Red No. 40 Aluminum Lake), Food Yellow No. 4 Aluminum Lake (FD & C Yellow No. 5 Aluminum Lake), Food Yellow No. 5 Aluminum Lake (FD & C Yellow No. 6 Aluminum Lake), Food Blue No. 1 Aluminum Lake (FD & C Blue No. 1 Aluminum Lake), and Food Blue No. 2 Aluminum Lake (FD & C Blue No. 2 Aluminum Lake).

The natural pigment derivatives include, for example, norbixin potassium. The natural synthetic pigments include, for example, β-carotene, riboflavin, and the like.

The Color Index Names mentioned above are the names established by American Association of Textile Chemists and Colorists. The FDA Names mentioned above are the names established by U.S. FDA (U.S. Food and Drug Administration). Pigments (materials) that can be used in the present embodiment have been specified by CAS Numbers, but the present invention should not be limited to these. As a matter of course, for example, pigments that can be used in the present embodiment include those pigments which have the same names as those of the pigments (materials) mentioned in the present embodiment but have different CAS Numbers because of being geometric isomers, stereoisomers, materials comprising isotopes, or salts thereof. If there are no isomers or the like, or if pigments (materials) that can be used are specified (limited), the materials (compounds) having the CAS Numbers indicated in the present embodiment can be used.

Examples of the natural food pigments include anthocyanin pigments, carotenoid pigments, quinone pigments, chlorophyll pigments, flavonoid pigments, betaine pigments, Monascus pigments, and other natural pigments originating from natural products. Examples of the anthocyanin pigments include red radish pigment, red cabbage pigment, red rice pigment, elderberry pigment, cowberry pigment, gooseberry pigment, cranberry pigment, salmon berry pigment, perilla pigment, sim blueberry pigment, strawberry pigment, dark sweet cherry pigment, cherry pigment, hibiscus pigment, huckleberry pigment, grape juice pigment, grape skin pigment, black currant pigment, blackberry pigment, blueberry pigment, plum pigment, whortleberry pigment, boysenberry pigment, mulberry pigment, purple potato pigment, purple corn pigment, Chinese purple potato pigment, raspberry pigment, red currant pigment, loganberry pigment, and other anthocyanin pigments. Examples of the carotenoid pigments include annatto pigment, gardenia yellow pigment, and other carotenoid pigments. Examples of the quinone pigments include cochineal pigment, lithospermum root pigment, lac pigment, and other quinone pigments. Examples of the flavonoid pigments include safflower yellow pigment, kaoliang pigment, onion pigment, and other flavonoid pigments. Examples of the betaine pigments include beet red pigment. Examples of the Monascus pigments include Monascus purpureus pigment, and Monascus yellow pigment. Examples of other pigments originating from natural products include turmeric pigment, Trichotomine pigment, gardenia red pigment, and spirulina blue pigment.

### (Discoloration inhibitor)

As mentioned above, the edible IJ ink of the present embodiment contains hydroxy acid salt having two or more carboxyl groups. The hydroxy acid salt functions as a discoloration inhibitor for Red No. 3 and thus has the effect of suppressing photolysis of Red No. 3. Thus, the edible IJ ink of the present embodiment can sufficiently suppress photodiscoloration or photofading of printed images and improve lightfastness, while maintaining the initial printed color (the color immediately after printing). Specifically, the edible IJ ink of the present embodiment may only need to contain at least one of citrate and malate as the hydroxy acid salt having two or more carboxyl groups. The edible IJ ink of the present embodiment contains at least one of citrate and malate, or may contain both of them.

The blending ratio of the hydroxy acid salt (citrate and/or malate) in the edible IJ ink of the present embodiment, i.e., the total content of the hydroxy acid salt, is preferred to be in the range of 0.5 mass% or more and 30 mass% or less with respect to the total mass of the ink. With this composition, the effect of the hydroxy acid salt as a discoloration inhibitor can be more reliably exerted to reliably improve lightfastness of the edible IJ ink of the present embodiment and to prevent undissolved residue of the hydroxy acid salt from remaining in the edible IJ ink when producing the ink. Herein, for example, if undissolved residue of the hydroxy acid salt cannot be visually recognized after the step of stirring a solvent and the hydroxy acid salt (e.g., the step of adding the hydroxy acid salt to a solvent at 25°C and stirring the mixture for 60 minutes) when producing the edible IJ ink, solubility of the hydroxy acid salt in the solvent is assumed to be secured.

In contrast, if the blending ratio of the hydroxy acid salt is less than 0.5 mass%, the effect of the hydroxy acid salt as a discoloration inhibitor for the ink can be reduced. If the content of the hydroxy acid salt exceeds 30 mass%, undissolved residue of the hydroxy acid salt could be left in the edible IJ ink in the stirring step when producing the ink.

### (Citrate)

Examples of citrate among the hydroxy acid salts to be contained as a discoloration inhibitor in the edible IJ ink of the present embodiment include isopropyl citrate, triethyl citrate, monopotassium citrate, tripotassium citrate, calcium citrate, sodium ferrous citrate, iron citrate and ammonium ferric citrate.

### (Malate)

Malate among the hydroxy acid salts to be contained as a discoloration inhibitor in the edible IJ ink of the present embodiment is sodium malate. The sodium malate may be, for example, sodium ferrous malate, disodium malate, or the like. Other than these, for example, isopropyl malate, diethyl malate, monopotassium malate, dipotassium malate, calcium malate, iron malate, ferric ammonium malate, or the like may be used as a discoloration inhibitor.

### (Solvent)

The edible IJ ink of the present embodiment may contain a solvent (dispersion medium) to dissolve (disperse) the specific tar pigment (Red No. 3), and contain the hydroxy acid salt as a discoloration inhibitor. The inkjet ink of the present embodiment may only need to contain at least one of water (e.g., purified water), ethanol, and propylene glycol, as a solvent. The propylene glycol can function as a wetting agent to prevent the ink from drying at the inkjet nozzles and impart sufficient intermittent resumability to the ink. The ethanol has high volatility and thus can improve transfer resistance (dryness) of the edible IJ ink. By adding these components to the solvent in addition to water, performance according to each component can be imparted to the edible IJ ink.

The blending ratios of the components of the solvent are not limited in the edible IJ ink of the present embodiment; however, if the solvent contains propylene glycol, the blending of the propylene glycol, i.e., the amount of the propylene glycol added, may be in the range of 1 mass% or more and 40 mass% or less with respect to the total mass of the ink. With this composition, sufficient intermittent resumability can be imparted to the ink.

If the solvent contains ethanol, the blending ratio of the ethanol, i.e., the amount of the ethanol added, may be in the range of 1 mass% or more and 55 mass% or less with respect to the total mass of the ink. With this composition, transfer resistance (dryness) of the edible IJ ink can be improved.

The edible IJ ink of the present embodiment may be formulated by adding solvent components other than those described above. Examples of the solvent components that can be added to the edible IJ ink of the present embodiment include glycerin, polyethyleneglycol 300 (having an average molecular weight of 300), 1-propanol, 2-propanol, and ethyl lactate. The blending ratios of the solvent components are not particularly limited; however, the ink may contain either of glycerin and polyethyleneglycol 300 in the range of 1 mass% or more and 40 mass% or less to prevent the ink from drying at the nozzles.

### (Lightfastness)

Hereinafter, lightfastness of the edible IJ ink of the present embodiment will be described in detail.

The edible IJ ink of the present embodiment may only need to have a color difference ΔE of 17 or less according to JIS Z 8781 before and after lightfastness testing of the printed image. The lightfastness testing herein refers to, for example, a test of comparing values of the color difference ΔE (based on JIS Z 8781), i.e., variation in chromaticity and optical color density, before and after visible light irradiation, for the printed image which is printed using the edible IJ ink of the present embodiment. Specifically, the color difference ΔE is measured before and after irradiation of a cumulative 1.2 million lux of visible light to the printed image and the values are compared to each other. For the visible light irradiation, a Xenon Weather-Ometer (Ci4000 manufactured by Toyo Seiki Seisaku-sho, Ltd.) is used, for example. The printed image used in the lightfastness testing is an image which is solid-printed on a tablet (e.g., film-coated tablet), for example, as a printing target.

The necessary lightfastness to be achieved as a result of forming a printed image on a pharmaceutical tablet, etc. (lightfastness practically necessary for a pharmaceutical tablet, etc.) substantially corresponds to the color difference ΔE of a printed image being 15 or less before and after visible light irradiation in the lightfastness testing of applying visible light of 1.2 million lux. Using the color difference ΔE of 15 or less as a practical criterion for lightfastness has been derived as a result of opinion tests conducted by the present inventors with medical professionals. However, since colors are clear due to use of Red No. 3 as a coloring agent in the edible IJ ink of the present embodiment, if the color difference ΔE of 17 or less is satisfied, the ink was determined to have sufficient lightfastness and evaluated to be above the pass level.

The edible IJ ink of the present embodiment contains Red No. 3 as a colorant, while addition of the hydroxy acid salt as a discoloration inhibitor can suppress photolysis of Red No. 3 on the tablet surface to sufficiently suppress photodiscoloration or photofading of the printed image. Thus, the color difference ΔE becomes less than 17 before and after lightfastness testing, and lightfastness of the edible IJ ink can be improved.

### (Internal-sizing resin)

The edible IJ ink of the present embodiment may contain an internal-sizing resin other than the above pigment and solvent. The internal-sizing resin that can be added to the edible IJ ink of the present embodiment may be an edible resin-like material in the form of a water-soluble powder, paste, or flakes which are capable of forming a coating on the surface of a tablet when dried following printing. Examples of the internal-sizing resin include polyvinyl alcohols (PVAs), hydroxypropyl celluloses (HPCs), hydroxypropyl methylcelluloses (HPMCs), polyvinylpyrrolidones (PVPs), high molecular weight polyethylene glycols (PEGs) such as polyethylene glycol 4000 or polyethylene glycol 1540, shellac resins, methacrylic acid copolymers (product name: Eudragit S100), maltodextrins, and erythritols.

### (Leveling agent)

The edible IJ ink of the present embodiment may contain a leveling agent other than the above pigment, solvent, and internal-sizing resin. The levelling agent that can be added to the edible IJ ink of the present embodiment may only need to be an edible and water-soluble surfactant. Examples of the levelling agent include polyglycerin fatty acid esters (e.g., Decaglyceryl distearate Q-182S or Decaglyceryl monolaurate Q-12S manufactured by Taiyo Kagaku Co.,Ltd.), sorbitan fatty acid esters (e.g., NIKKOLSL-10 manufactured by Nikko Chemicals Co., Ltd.), sucrose fatty acid esters (e.g., DK Ester F-110 manufactured by DKS Co. Ltd.), and polysorbates (Emazole S-120 series manufactured by Kao Corporation).

### (Printing method)

The printing method using the edible IJ ink of the present embodiment is not particularly limited, but printing using an inkjet device, such as a commercially available inkjet printer, may be used. Therefore, the edible IJ ink of the present embodiment has a wide range of applications and is very useful. For example, the edible IJ ink of the present embodiment can be printed using a drop-on-demand inkjet device including a piezoelectric element (piezoelectric ceramic) as an actuator, or inkjet devices of other types.

Examples of the drop-on-demand inkjet device include a thermal-inkjet type device ejecting an IJ ink using water vapor pressure generated by instantaneously heating a micro-heating element to a high temperature (of 200°C to 300°C), an electrostatic-type device ejecting an IJ ink by electrostatically vibrating an actuator, and an ultrasonic-type device using an ultrasonic cavitation phenomenon. If the edible IJ ink of the present embodiment has charging performance, a continuous injection-type device may be used.

### [Tablet]

The edible IJ ink of the present embodiment may be used for printing characters or an image on the surfaces of tablets, for example, using the above printing methods. In other words, the tablet according to the present embodiment may have a printed part, i.e., a printed image, which is printed using the edible IJ ink of the present embodiment. As long as the edible IJ ink according to the present embodiment is used, lightfastness can be improved in the printed images (e.g., printed image 3), for example, provided on the surfaces of pharmaceutical tablets using inkjet printing methods. The following description explains a composition of a tablet having a printed image printed using the edible IJ ink of the present embodiment.

The tablet of the present embodiment is, for example, a pharmaceutical tablet. Examples of the pharmaceutical tablet herein include film-coated tablets having an outermost surface on which a water-soluble surface layer is formed, as well as uncoated tablets (bare tablets), sugar-coated tablets, enteric tablets, and orally disintegrating tablets.

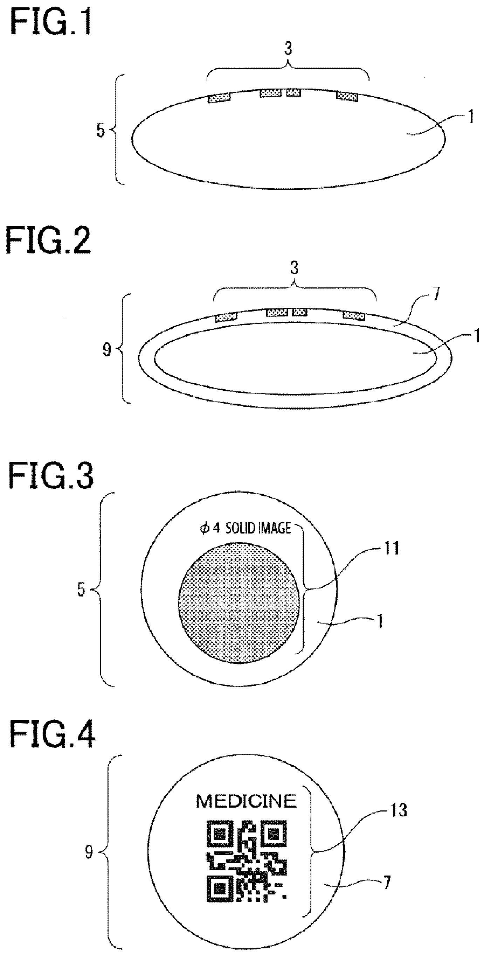

Fig. 1 is a schematic cross-sectional view illustrating an example of a pharmaceutical tablet (uncoated tablet) having a print (characters or image). Fig. 1 shows uncoated tablet printed matter 5, in cross-sectional view, with a printed image 3, such as characters, printed on the upper surface of a substrate 1 of the tablet.

Fig. 2 is a schematic cross-sectional view illustrating an example of a pharmaceutical tablet (film-coated (FC) tablet) provided with a print (characters or image) thereon. Fig. 2 shows film-coated tablet printed matter 9, in cross-sectional view, with a printed image 3, such as characters, printed on the upper surface of the substrate 1 of the tablet, on the surface of which a film coating layer 7 is formed.

In the present embodiment, a solid image may be printed as an uncoated tablet printed image 11 as shown in Fig. 3, or a two-dimensional barcode may be printed as a film-coated tablet printed image 13 as shown in Fig. 4.

The pharmaceutical table may contain unlimited active ingredients. Examples of the active ingredients include, but are not limited thereto, substances effective for preventing or treating various diseases (e.g., substances having a sleep-inducing effect, tranquilizer activity, antibacterial activity, antihypertensive effect, anti-angina activity, analgesic effect, antiinflammatory activity, tranquilizing effect, diabetes treatment activity, diuretic effect, anticholinergic activity, anti-hyperacidity effect, antiepileptic effect, ACE inhibitory activity, β-receptor antagonist or agonist activity, anesthetic action, appetite suppressant action, antiarrhythmic effect, antidepressant effect, anticoagulant activity, antidiarrheal effect, antihistamine activity, antimalarial effect, antitumor activity, immunosuppressive activity, antiparkinsonian effect, antipsychotic effect, antiplatelet activity, and antihyperlipidemic effect), substances having a scavenging effect, and substances having a scent or a deodorant action.

In the tablet of the present embodiment, carriers which are compatible from the perspective of usage with the active ingredients may be mixed as necessary. For example, pharmaceutical tablets may comprise carriers which are compatible from the pharmaceutical perspective. As carriers compatible from the pharmaceutical perspective, various organic or inorganic carriers, which are commonly used as pharmaceutical materials, may be appropriately mixed, such as excipients, lubricants, binders, disintegrants, and thickeners. As necessary, additives such as antiseptics, antioxidants, colorants, or sweeteners may be used.

Although the present embodiment has been described taking an example of a pharmaceutical tablet as a tablet, tablet is not limited to this tablet of the present invention. Printing targets of the edible IJ ink of the present embodiment are not particularly limited. For example, the edible IJ ink of the present embodiment may be printed on the surfaces of various tablets, including tablets to be administered to non-human animals (e.g., pets, livestock, poultry, etc.), or tablets of feed, fertilizers, and cleaning agents, and food tablets such as soda-pop flavored confectionery tablets and supplement tablets. The edible IJ ink of the present embodiment does not particularly limit the size of the printing target, but may be applied to tablets of various sizes.

### (Advantageous effects of the present embodiment)

(1) The edible IJ ink according to the present embodiment contains Red No. 3, and hydroxy acid salt having two or more carboxyl groups.
   With this composition, photodiscoloration or photofading of printed images can be sufficiently suppressed and lightfastness of the edible IJ ink can be improved, compared to the conventional art.
(2) The edible IJ ink according to the present embodiment contains at least one of citrate and malate as the hydroxy acid salt.
   With this composition, photodiscoloration or photofading of printed images can be sufficiently suppressed and lightfastness of the edible IJ ink can be reliably improved, compared to the conventional art.
(3) In the edible IJ ink according to the present embodiment, the amount of Red No. 3 added may be in the range of 2.5 mass% or more and 10 mass% or less with respect to the total mass of the ink.
   With this composition, solubility of Red No. 3 in the solvent can be secured, while lightfastness of the edible IJ ink can be further improved, compared to the conventional art.
(4) In the edible IJ ink according to the present embodiment, the amount of the hydroxy acid salt added may be in the range of 0.5 mass% or more and 30 mass% or less with respect to the total mass of the ink.
   With this composition, solubility of the hydroxy acid salt in the solvent can be secured, while lightfastness of the edible IJ ink can be improved, compared to the conventional art.
(5) The hydroxy acid salt contained in the edible IJ ink according to the present embodiment is at least one of isopropyl citrate, triethyl citrate, monopotassium citrate, tripotassium citrate, calcium citrate, sodium ferrous citrate, iron citrate, ammonium ferric citrate and sodium malate.
   With this composition, lightfastness of the edible IJ ink can be reliably improved, compared to the conventional art.
(6) The edible IJ ink according to the present embodiment may only need to have a color difference ΔE of 17 or less according to JIS Z 8781 before and after lightfastness testing of the printed image.
   With this composition, photodiscoloration or photofading of printed images can be reliably suppressed and lightfastness of the edible IJ ink can be reliably improved, compared to the conventional art.
(7) The tablet according to the present embodiment is provided with the printed image 3 (one example of the printed part) which is printed using the edible IJ ink described above.
   With this configuration, photodiscoloration or photofading of the printed image 3, etc. directly printed on the surface, etc. of the tablet can be sufficiently suppressed and lightfastness of the printed image 3 can be sufficiently improved, compared to the conventional art. Furthermore, edibility can also be imparted to the printed image portions on the surface of the tablet.
(8) The tablet according to the present embodiment may be a pharmaceutical tablet.
   Using the edible IJ ink, photodiscoloration or photofading can be sufficiently suppressed in pharmaceutical tablets, and the occurrence of incorrect dispensing or dosing can be reduced, which would occur due to deterioration in visibility of the printed images.

### [Examples]

The present invention will be further described in detail using examples; however the present invention should not be limited to these examples.

### <Example 1>

A procedure of preparing an edible IJ ink of Example 1 will be described.

### [Composition of edible IJ ink]

First, a printing ink was prepared. Edible IJ inks contain components such as a pigment (colorant), solvent, and discoloration inhibitor. Regarding the preparation procedure, first, water, propylene glycol, and ethanol were mixed with each other to obtain a mixed solvent. Next, a discoloration inhibitor and a colorant were added to the mixed solvent at 25°C, followed by stirring for a predetermined period of time (e.g., 60 min). Thus, an ink of the present example was prepared. The individual components will be specifically described below.

In the present example, tripotassium citrate was added to the solvent as the discoloration inhibitor to obtain a transparent base liquid. Next, Red No. 3, i.e., a food dye, as a colorant was added to the transparent base liquid, followed by stirring for about 1 hour, to obtain an edible IJ ink of Example 1. With respect to the total mass of the edible IJ ink of Example 1, the blending ratio of Red No. 3 as a colorant was 4.0 mass% and, in the solvent, the blending ratio of purified water was 59.5 mass%, that of propylene glycol was 28.0 mass%, and that of ethanol was 6.5 mass%. The blending ratio of tripotassium citrate as a discoloration inhibitor was 2.0 mass% with respect to the total mass of the edible IJ ink.

### <Example 2>

The components of the solvent (mixed solvent) were the same as in Example 1, but the blending ratio of purified water was 57.5 mass% with respect to the total mass of the ink. The blending ratio of tripotassium citrate as a discoloration inhibitor was 4.0 mass% with respect to the total mass of the edible ink. Except for these points, an edible IJ ink of Example 2 was obtained as in Example 1.

### <Example 3>

The components of the solvent (mixed solvent) were the same as in Example 1, but the blending ratio of purified water was 51.5 mass% with respect to the total mass of the ink. The blending ratio of tripotassium citrate as a discoloration inhibitor was 10.0 mass% with respect to the total mass of the edible IJ ink. Except for these points, an edible IJ ink of Example 3 was obtained as in Example 1.

### <Example 4>

The components of the solvent (mixed solvent) were the same as in Example 1, but the blending ratio of purified water was 31.5 mass% with respect to the total mass of the ink. The blending ratio of tripotassium citrate as a discoloration inhibitor was 30.0 mass% with respect to the total mass of the edible IJ ink. Except for these points, an edible IJ ink of Example 4 was obtained as in Example 1.

### <Example 5>

The components of the solvent (mixed solvent) were the same as in Example 1, but the blending ratio of purified water was 61.0 mass% with respect to the total mass of the ink. The blending ratio of tripotassium citrate as a discoloration inhibitor was 0.5 mass% with respect to the total mass of the edible IJ ink. Except for these points, an edible IJ ink of Example 5 was obtained as in Example 1.

### <Example 6>

The components of the solvent (mixed solvent) were the same as in Example 1, but the blending ratio of purified water was 53.5 mass% with respect to the total mass of the ink. The blending ratio of Red No. 3 was 10.0 mass% with respect to the total mass of the edible IJ ink. Except for these points, an edible IJ ink of Example 6 was obtained as in Example 1.

### <Example 7>

The components of the solvent (mixed solvent) were the same as in Example 1, but the blending ratio of purified water was 57.0 mass% with respect to the total mass of the ink. The blending ratio of Red No. 3 was 2.5 mass% with respect to the total mass of the edible IJ ink. The blending ratio of tripotassium citrate as a discoloration inhibitor was 6.0 mass% with respect to the total mass of the edible IJ ink. Except for these points, an edible IJ ink of Example 7 was obtained as in Example 1.

### <Example 8>

The components of the solvent (mixed solvent) were the same as in Example 1, but the blending ratio of purified water was 61.2 mass% with respect to the total mass of the ink. The blending ratio of tripotassium citrate as a discoloration inhibitor was 0.3 mass% with respect to the total mass of the edible IJ ink. Except for these points, an edible IJ ink of Example 8 was obtained as in Example 1.

### <Example 9>

The components of the solvent (mixed solvent) and the blending ratios of the components were the same as in Example 2. The blending ratio of tripotassium citrate as a discoloration inhibitor was 6.0 mass% with respect to the total mass of the edible IJ ink. The blending ratio of Red No. 3 was 2.0 mass% with respect to the total mass of the edible IJ ink. Except for these points, an edible IJ ink of Example 9 was obtained as in Example 1.

### <Example 10>

The components of the solvent (mixed solvent) and the blending ratios of the components were the same as in Example 2. Isopropyl citrate was added as a discoloration inhibitor at a blending ratio of 4.0 mass% with respect to the total mass of the edible IJ ink. Except for these points, an edible IJ ink of Example 10 was obtained as in Example 1.

### <Example 11>

The components of the solvent (mixed solvent) and the blending ratios of the components were the same as in Example 2. Triethyl citrate was added as a discoloration inhibitor at a blending ratio of 4.0 mass% with respect to the total mass of the edible IJ ink. Except for these points, an edible IJ ink of Example 11 was obtained as in Example 1.

### <Example 12>

The components of the solvent (mixed solvent) and the blending ratios of the components were the same as in Example 2. Monopotassium citrate was added as a discoloration inhibitor at a blending ratio of 4.0 mass% with respect to the total mass of the edible IJ ink. Except for these points, an edible IJ ink of Example 12 was obtained as in Example 1.

### <Example 13>

The components of the solvent (mixed solvent) and the blending ratios of the components were the same as in Example 2. Calcium citrate was added as a discoloration inhibitor at a blending ratio of 4.0 mass% with respect to the total mass of the edible IJ ink. Except for these points, an edible IJ ink of Example 13 was obtained as in Example 1.

### <Example 14>

The components of the solvent (mixed solvent) and the blending ratios of the components were the same as in Example 2. Sodium ferrous citrate was added as a discoloration inhibitor at a blending ratio of 4.0 mass% with respect to the total mass of the edible IJ ink. Except for these points, an edible IJ ink of Example 14 was obtained as in Example 1.

### <Example 15>

The components of the solvent (mixed solvent) and the blending ratios of the components were the same as in Example 2. Iron citrate was added as a discoloration inhibitor at a blending ratio of 4.0 mass% with respect to the total mass of the edible IJ ink. Except for these points, an edible IJ ink of Example 15 was obtained as in Example 1.

### <Example 16>

The components of the solvent (mixed solvent) and the blending ratios of the components were the same as in Example 2. Ferric ammonium citrate was added as a discoloration inhibitor at a blending ratio of 4.0 mass% with respect to the total mass of the edible IJ ink. Except for these points, an edible IJ ink of Example 16 was obtained as in Example 1.

### <Example 17> (Reference Example)

The components of the solvent (mixed solvent) and the blending ratios of the components were the same as in Example 2. Sodium dihydrogen citrate was added as a discoloration inhibitor at a blending ratio of 4.0 mass% with respect to the total mass of the edible IJ ink. Except for these points, an edible IJ ink of Example 17 was obtained as in Example 1.

### <Example 18> (Reference Example)

The components of the solvent (mixed solvent) and the blending ratios of the components were the same as in Example 2. Disodium citrate was added as a discoloration inhibitor at a blending ratio of 4.0 mass% with respect to the total mass of the edible IJ ink. Except for these points, an edible IJ ink of Example 18 was obtained as in Example 1.

### <Example 19> (Reference Example)

The components of the solvent (mixed solvent) and the blending ratios of the components were the same as in Example 2. Trisodium citrate was added as a discoloration inhibitor at a blending ratio of 4.0 mass% with respect to the total mass of the edible IJ ink. Except for these points, an edible IJ ink of Example 19 was obtained as in Example 1.

### <Example 20>

The components of the solvent (mixed solvent) and the blending ratios of the components were the same as in Example 2. DL-sodium malate was added as a discoloration inhibitor at a blending ratio of 4.0 mass% with respect to the total mass of the edible IJ ink. Except for these points, an edible IJ ink of Example 20 was obtained as in Example 1.

### <Comparative Example 1>

The components of the solvent (mixed solvent) were the same as in Example 1, but the blending ratio of purified water was 61.5 mass% with respect to the total mass of the ink. No discoloration inhibitor was added. Except for these points, an edible IJ ink of Comparative Example 1 was obtained as in Example 1.

### <Comparative Example 2>

The components of the solvent (mixed solvent) and the blending ratios of the components were the same as in Example 2. Citric acid was added as a discoloration inhibitor at a blending ratio of 4.0 mass% with respect to the total mass of the edible IJ ink. Except for these points, an edible IJ ink of Comparative Example 2 was obtained as in Example 1.

### <Comparative Example 3>

The components of the solvent (mixed solvent) and the blending ratios of the components were the same as in Example 2. DL-malic acid was added as a discoloration inhibitor at a blending ratio of 4.0 mass% with respect to the total mass of the edible IJ ink. Except for these points, an edible IJ ink of Comparative Example 3 was obtained as in Example 1.

### <Comparative Example 4>

The components of the solvent (mixed solvent) and the blending ratios of the components were the same as in Example 2. Red No. 102 was added as a colorant instead of Red No. 3 at a blending ratio of 4.0 mass% with respect to the total mass of the edible IJ ink. Except for these points, an edible IJ ink of Comparative Example 2 was obtained as in Example 1.

### <Comparative Example 5>

The components of the solvent (mixed solvent) and the blending ratios of the components were the same as in Example 2. Blue No. 1 was added as a colorant instead of Red No. 3 at a blending ratio of 4.0 mass% with respect to the total mass of the edible IJ ink. Except for these points, an edible IJ ink of Comparative Example 5 was obtained as in Example 1.

### <Comparative Example 6>

The components of the solvent (mixed solvent) were the same as in Example 1, but the blending ratio of purified water was 59.0 mass% with respect to the total mass of the ink. Copper chlorophyllin sodium was added as a colorant instead of Red No. 3 at a blending ratio of 2.5 mass% with respect to the total mass of the edible IJ ink. Except for these points, an edible IJ ink of Comparative Example 6 was obtained as in Example 1.

### <Comparative Example 7>

The components of the solvent (mixed solvent) and the blending ratios of the components were the same as in Example 2. Tea extract was added as a discoloration inhibitor at a blending ratio of 4.0 mass% with respect to the total mass of the edible IJ ink. Except for these points, an edible IJ ink of Comparative Example 7 was obtained as in Example 1.

### <Comparative Example 8>

The components of the solvent (mixed solvent) and the blending ratios of the components were the same as in Example 2. 5-Caffeoyl Quinic Acid (product name: Caffenol P-100) was added as a discoloration inhibitor at a blending ratio of 4.0 mass% with respect to the total mass of the edible IJ ink. Except for these points, an edible IJ ink of Comparative Example 8 was obtained as in Example 1.

### <Comparative Example 9>

The components of the solvent (mixed solvent) were the same as in Example 1, but the blending ratio of purified water was 59.5 mass% with respect to the total mass of the ink. Extracted vitamin E was added as a discoloration inhibitor at a blending ratio of 2.0 mass% with respect to the total mass of the edible IJ ink. Except for these points, an edible IJ ink of Comparative Example 9 was obtained as in Example 1.

### <Comparative Example 10>

The components of the solvent (mixed solvent) and the blending ratios of the components were the same as in Example 2. Phytic acid was added as a discoloration inhibitor at a blending ratio of 4.0 mass% with respect to the total mass of the edible IJ ink. Except for these points, an edible IJ ink of Comparative Example 10 was obtained as in Example 1.

### <Comparative Example 11>

The components of the solvent (mixed solvent) and the blending ratios of the components were the same as in Example 2. Vitamin C was added as a discoloration inhibitor at a blending ratio of 4.0 mass% with respect to the total mass of the edible IJ ink. Except for these points, an edible IJ ink of Comparative Example 11 was obtained as in Example 1.

Each of the inks of Examples 1 to 20 and Comparative Examples 1 to 11 was passed through a membrane filter to remove solid foreign matter from the liquid. Specifically, each of the inks was passed once through a membrane filter (cellulose acetate film) having a pore size of 5.0 µm, and subsequently, was passed once through a membrane filter (cellulose acetate film) having a pore size of 0.8 µm to obtain a purified ink.

### <Evaluation>

For the inks of the above examples and comparative examples, solubility and lightfastness were evaluated using the following method. The results of evaluation are shown in the following Tables 1 and 2 together with the ink compositions of the examples and comparative examples.

### (Lightfastness testing)

Using a piezoelectric ceramic-driven drop-on-demand inkjet head having a print resolution of 600 dpi in the main scanning direction and 600 dpi in the sub-scanning direction (conveyance direction of the recording medium such as a tablet) and having 2,656 nozzles in total, an image was printed on the following tablet using the inks of Examples 1 to 20 and Comparative Examples 1 to 11 at 6 pl per drop.

The tablet as a printing target was a film-coated tablet for testing (base: conditioned starch, coating agent: mixture of 70% hydroxypropyl methylcellulose and 30% titanium oxide, diameter: 6.5 mm). The printed image was a circular solid image (diameter: 4.0 mm). Thus, film-coated tablet printed matter was obtained.

Using a xenon weather meter (Ci4000 manufactured by Toyo Seiki Seisaku-sho, Ltd.), a cumulative 1.2 million lux of visible light was applied to the above film-coated tablet printed matter of the examples and comparative examples of which the chromaticity and optical color density were measured. Using a spectrophotometer, chromaticity and optical color density were measured for the film-coated tablet printed matter irradiated with visible light, and a comparison was made between values of the color difference ΔE (based on JIS Z 8781), i.e., variation in chromaticity and optical color density, before and after the visible light irradiation. The comparison results are shown in Tables 1 and 2. As described above, regarding the change of color before and after visible light irradiation, the present inventors have found that if the color difference ΔE according to JIS Z 8781 was 17 or less (ΔE≤17), the edible IJ ink was determined to have sufficient lightfastness to withstand practical use and evaluated to be above the pass level. Specific evaluation criteria for light resistance were as follows.

Very good: ΔE was 13 or less (ΔE≤13).

Good: ΔE was 15 or less (ΔE≤15).

Fair: ΔE was 17 or less (ΔE≤17).

Poor: ΔE exceeded 17 (ΔE>17).

### (Solubility testing)

In the step of producing the edible IJ ink of the examples and comparative examples, the colorant and the discoloration inhibitor were added to the solvent at 25°C, followed by stirring for 60 minutes. After that, the presence or absence of undissolved residue of the colorant and discoloration inhibitor was visually inspected. The evaluation criteria were as follows.

Very good: Undissolved residue of neither the colorant nor the discoloration inhibitor was found.

Poor: Undissolved residue of at least one of the colorant and discoloration inhibitor was found.

The edible IJ ink composition and the results of evaluation of the examples are shown in Examples 17, 18 and 19 are Reference Examples not according to the invention. The edible IJ ink composition and the results of evaluation of the comparative examples are shown in Table 2. In Tables 1 and 2, blank cells or cells with "-" indicate that the substances were not used. In Tables 1 and 2, the film-coated tablet is abbreviated as FC tablet. In Table 2, cells with "-" indicate that no lightfastness testing was conducted for the comparative examples.

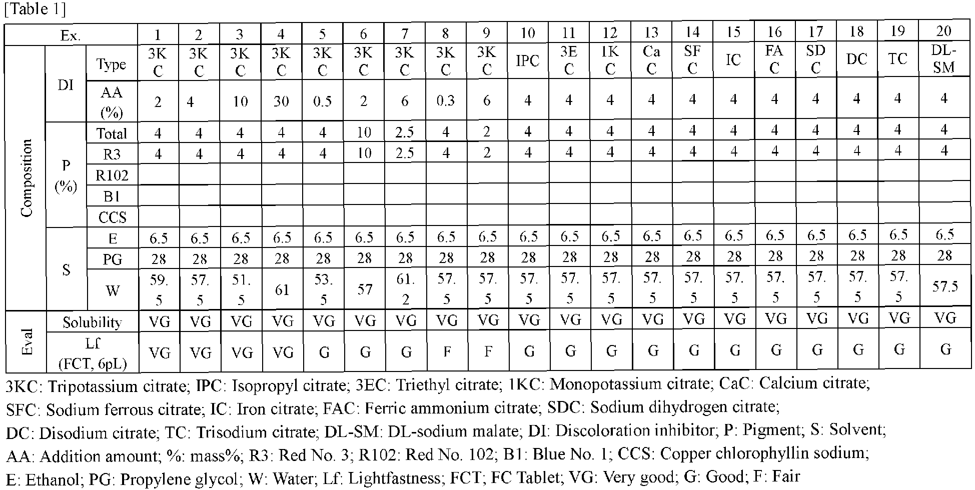

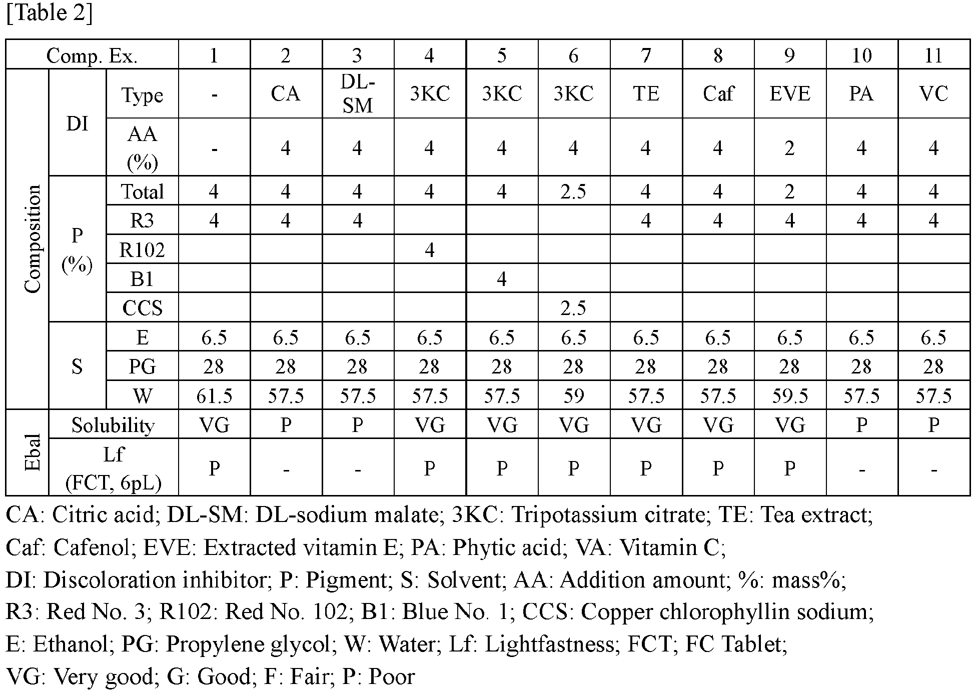

As shown in Table 1, the edible IJ inks of Examples 1 to 20 were all evaluated to be very good in solubility testing, specifically, they were found to leave no undissolved residue of the colorant and discoloration inhibitor when produced and secure solubility. However, of the edible IJ inks of Comparative Examples 1 to 11, the edible IJ inks of Comparative Examples 2, 3, 10 and 11 were evaluated to be poor in solubility testing, specifically, they left undissolved residue of the colorant and discoloration inhibitor. For the edible IJ inks of comparative examples which were below the pass level (poor) in solubility testing, lightfastness testing was not conducted.

As shown in Table 1, the film-coated tablet printed matter of Examples 1 to 20 all showed a color difference ΔE of 17 or less (ΔE≤17) before and after visible light irradiation, in lightfastness testing. In other words, the edible IJ inks of Examples 1 to 20 were found to sufficiently suppress photodiscoloration or photofading of the printed image and be imparted with good lightfastness. In contrast, as shown in Table 2, the edible IJ inks of Comparative Examples 2, 3, 10 and 11 were evaluated to be poor in solubility, i.e., below the pass level, and no lightfastness testing was conducted. The film-coated tablet printed matter using the edible IJ inks of Comparative Examples 1 and 4 to 9 all showed a color difference ΔE exceeding 17 (ΔE>17) before and after visible light irradiation, in lightfastness testing. In other words, the edible IJ inks of Comparative Examples 1 to 11 were found to be unable to sufficiently suppress photodiscoloration or photofading of the printed image and be insufficient in lightfastness.

From the results of the lightfastness testing, it was found that, as long as the edible IJ ink contains Red No. 3 and a hydroxy acid salt having two or more carboxyl groups, photodiscoloration or photofading could be sufficiently suppressed and lightfastness was improved.

### [Reference Signs List]

- 1: Substrate of tablet
- 3: Printed image
- 5: Uncoated tablet printed matter
- 7: Film coating layer
- 9: Film-coated tablet printed matter
- 11: Uncoated tablet printed image (solid image)
- 13: Film-coated tablet printed image (two-dimensional barcode)

## Claims

1. An edible inkjet ink comprising Red No. 3, and hydroxy acid salt having two or more carboxyl groups,
wherein the hydroxy acid salt is at least one of isopropyl citrate, triethyl citrate, monopotassium citrate, tripotassium citrate, calcium citrate, sodium ferrous citrate, iron citrate, ammonium ferric citrate, and sodium malate.

2. The edible inkjet ink according to claim 1, comprising at least one of citrate and malate as the hydroxy acid salt.

3. The edible inkjet ink according to claim 1 or 2, wherein
a blending ratio of the Red No. 3 is in a range of 2.5 mass% or more and 10 mass% or less with respect to a total mass of the ink.

4. The edible inkjet ink according to any one of claims 1 to 3, wherein
a blending ratio of the hydroxy acid salt is in a range of 0.5 mass% or more and 30 mass% or less with respect to a total mass of the ink.

5. The edible inkjet ink according to any one of claims 1 to 4, wherein
a color difference ΔE according to JIS Z 8781 is 17 or less before and after lightfastness testing of a printed image.

6. A tablet comprising a printed part that is printed using the edible inkjet ink according to any one of claims 1 to 5.

7. The tablet according to claim 6, wherein the tablet is a pharmaceutical tablet.

## Patentansprüche

1. Essbare Tintenstrahltinte, die Rot Nr. 3 und Hydroxysäuresalz, das zwei oder mehr Carboxylgruppen aufweist, umfasst,
wobei das Hydroxysäuresalz mindestens eines ist von Isopropylcitrat, Triethylcitrat, Monokaliumcitrat, Trikaliumcitrat, Calciumcitrat, Natrium-Eisen(II)-citrat, Eisencitrat, Ammonium-Eisen(III)-citrat und Natriummalat.

2. Essbare Tintenstrahltinte nach Anspruch 1, die mindestens eines von Citrat und Malat als das Hydroxysäuresalz umfasst.

3. Essbare Tintenstrahltinte nach Anspruch 1 oder 2, wobei
ein Mischungsverhältnis des Rot Nr. 3 in einem Bereich von 2,5 Massen-% oder mehr und 10 Massen-% oder weniger ist, bezogen auf eine Gesamtmasse der Tinte.

4. Essbare Tintenstrahltinte nach einem der Ansprüche 1 bis 3, wobei
ein Mischungsverhältnis des Hydroxysäuresalzes in einem Bereich von 0,5 Massen-% oder mehr und 30 Massen-% oder weniger ist, bezogen auf eine Gesamtmasse der Tinte.

5. Essbare Tintenstrahltinte nach einem der Ansprüche 1 bis 4, wobei
ein Farbunterschied ΔE nach JIS Z 8781 vor und nach der Lichtechtheitsprüfung eines gedruckten Bildes 17 oder weniger ist.

6. Tablette, die einen bedruckten Teil umfasst, der unter Verwendung der essbaren Tintenstrahltinte nach einem der Ansprüche 1 bis 5 bedruckt ist.

7. Tablette nach Anspruch 6, wobei die Tablette eine pharmazeutische Tablette ist.

## Revendications

1. Encre comestible pour jet d'encre comprenant Red n° 3, et un sel d'un hydroxyacide ayant deux groupes carboxyle ou plus,
dans laquelle le sel d'hydroxyacide est au moins l'un parmi le citrate d'isopropyle, le citrate de triéthyle, le citrate monopotassique, le citrate tripotassique, le citrate de calcium, le citrate ferreux de sodium, le citrate de fer, le citrate ferrique d'ammonium et le malate de sodium.

2. Encre comestible pour jet d'encre selon la revendication 1, comprenant au moins l'un parmi un citrate et un malate en tant que sel d'hydroxyacide.

3. Encre comestible pour jet d'encre selon la revendication 1 ou 2, dans laquelle
un rapport de mélange du Red n° 3 est dans une plage de 2,5 % en masse ou plus et 10 % en masse ou moins par rapport à une masse totale de l'encre.

4. Encre comestible pour jet d'encre selon l'une quelconque des revendications 1 à 3, dans laquelle
un rapport de mélange du sel d'hydroxyacide est dans une plage de 0,5 % en masse ou plus et 30 % en masse ou moins par rapport à une masse totale de l'encre.

5. Encre comestible pour jet d'encre selon l'une quelconque des revendications 1 à 4, dans laquelle
une différence de couleur ΔE selon la norme JIS Z 8781 est de 17 ou moins avant et après le test de résistance à la lumière d'une image imprimée.

6. Comprimé comprenant une partie imprimée qui est imprimée en utilisant l'encre comestible pour jet d'encre selon l'une quelconque des revendications 1 à 5.

7. Comprimé selon la revendication 6, le comprimé étant un comprimé pharmaceutique.
